# EUROPÄISCHE PATENTSCHRIFT

(11) **EP 2 277 466 B1**
(45) Veröffentlichungstag und Bekanntmachung des Hinweises auf die Patenterteilung: **15.02.2012**
(21) Anmeldenummer: 09009581.1
(22) Anmeldetag: 23.07.2009
(51) Int. Cl.: A61B 17/70, A61B 17/86

(54) **Knochenverankerungselement**
Bone anchoring element
Elément d'ancrage d'os

(43) Veröffentlichungstag der Anmeldung: 26.01.2011
(73) Patentinhaber: Böhm, Heinrich, Dr., 99425 Weimar (DE)
(72) Erfinder: Böhm, Heinrich, Dr., 99425 Weimar (DE); Wenzler, Klaus, 78665 Frittlingen (DE); Burger, Andreas, 78532 Tuttlingen (DE); Widmaier, Gerd, 78532 Tuttlingen (DE)
(74) Vertreter: Patentanwälte Westphal, Mussgnug & Partner

(56) Entgegenhaltungen:
- WO-A1-2004/103194
- WO-A1-2009/058318
- WO-A2-02/22030
- WO-A2-2006/041532
- FR-A1- 2 794 637
- US-A1- 2006 173 454
- US-A1- 2008 004 625
- US-A1- 2008 009 862

## Beschreibung

Die Erfindung betrifft ein Knochenverankerungselement gemäß dem Oberbegriff des Patentanspruchs 1. Ein derartiges Knochenverankerungselement ist aus der WO-A-2006/041532 bekannt.

Bekannt sind Knochenverankerungselemente mit einer einen Schaft und einen abschnittsweise kugeligen Kopf aufweisenden Schraube und einem eine Längsachse aufweisenden Stabaufnahmeelement zum Verbinden der Schraube mit einem stabförmigen Element, wobei der Schaft einen Knochengewindeabschnitt und der Kopf einen Gewindeabschnitt aufweist und wobei das Stabaufnahmeelement quer zur Längsachse einen Kanal zur Aufnahme des stabförmigen Elements und ein Halterelement zur Aufnahme des Kopfs der Schraube aufweist. Derartige Knochenverankerungselemente werden insbesondere in der Wirbelsäulenchirurgie eingesetzt. Ein derartiges Knochenverankerungselement zeigt beispielsweise die US 2008/0009862A1. Das Halteelement gemäß der US 2008/0009862A1 ist dabei als zylindrische Buchse ausgebildet, welche von einer Seite her in das Stabaufnahmeelement eingesetzt und mittels eines Halterings fixiert wird. Der kugelige Kopf der Schraube wird über ein auf der Außenseite des Kopfes der Schraube angebrachtes Gewinde und ein in dem Haltering angeordnetes Gewinde in den Innenraum des Stabaufnahmeelements eingeschraubt, wo er zwischen dem Haltering und dem Halteelement zum Liegen kommt.

Als Stand der Technik werden die US 2008/009862 A1, WO 2009/058318 A1, WO 2006/041532 A2, US 2006/173454 A1, WO 2004/103194 A1, US 2008/004625 A1, WO 02/22030 A2 und FR 2 794 637 A1 genannt.

Die Aufgabe der Erfindung besteht darin, ein Knochenverankerungselement weiter zu bilden, um ein flexibleres Knochenverankerungselement bereitzustellen.

Die Aufgabe wird gelöst durch ein Knochenverankerungselement mit den Merkmalen des Patentanspruchs 1.

Vorteilhafte Ausgestaltungen und Weiterbildungen der Erfindung sind in den Unteransprüchen angegeben.

Das erfindungsgemäße Knochenverankerungselement mit einer einen Schaft und einen abschnittsweise kugeligen Kopf aufweisenden Schraube und einem eine Längsachse aufweisenden Stabaufnahmeelement zum Verbinden der Schraube mit einem stabförmigen Element, wobei der Schaft einen Knochengewindeabschnitt und der Kopf einen Gewindeabschnitt aufweist und das Stabaufnahmeelement quer zur Längsachse einen Kanal zur Aufnahme des stabförmigen Elements und ein Halteelement zur Aufnahme des Kopfes der Schraube aufweist, zeichnet sich dadurch aus, dass die Innenfläche des Halteelements abschnittsweise hohlkugelig ausgebildet ist und den Kopf der Schraube zumindest teilweise umgreift. Unter einem teilweisen Umgreifen wird dabei ein Umgreifen in Längsrichtung des Kopfes der Schraube verstanden. Insbesondere ist unter einem teilweisen Umgreifen zu verstehen, dass die Innenfläche des Halteelements sowohl oberhalb als auch unterhalb eines größten Durchmessers des Kopfes der Schraube quer zur Längsachse der Schraube gemessen an dem Kopf der Schraube zur Anlage kommen kann. Die Schraube wird bei einem teilweisen Umgreifen somit insbesondere durch das Halteelement in axialer Richtung gehalten. Durch die hohlkugelartige Ausbildung der Innenfläche des Halteelements ergibt sich eine zuverlässige Führung, welche eine flexible Positionierung des Schraubenkopfes in dem Stabaufnahmeelement ermöglicht.

Erfindungsgemäß weist das Halteelement eine erste Öffnung auf, durch welche der Kopf der Schraube mittels des Gewindeabschnitts des Kopfes in das Halteelement eindrehbar ist, so dass auf diese Weise der Kopf der Schraube innerhalb des Halteelements positioniert werden kann.

Vorzugsweise schließt sich an die erste Öffnung auf der Innenfläche des Halteelements ein Gewindeabschnitt an, um das Eindrehen des Kopfes der Schraube in das Halteelement zu vereinfachen.

Erfindungsgemäß ist die Außenfläche des Halteelements abschnittsweise konisch ausgebildet, um eine zuverlässige Positionierung des Halteelements in dem Stabaufnahmeelement zu ermöglichen.

Vorzugsweise ist auf der Außenfläche des Halteelements zumindest abschnittsweise umlaufend wenigstens eine Ausnehmung angeordnet. Besonders bevorzugt ist auf der Außenfläche des Halteelements umlaufend ein Freistich angeordnet. Auf diese Weise wird das Halteelement in dem Bereich der Ausnehmung federnd ausgestaltet, um insbesondere das Eindrehen des Kopfes der Schraube zu erleichtern.

Gemäß einer bevorzugten Ausführungsform der Erfindung weist das Halteelement einen zylindrischen Abschnitt auf, über welchen das Halteelement in dem Stabaufnahmeelement in axialer Richtung exakt positioniert ausgerichtet werden kann.

Gemäß einer vorteilhaften Weiterbildung der Erfindung weist das Halteelement, insbesondere der zylindrische Abschnitt des Halteelements, in Längsrichtung wenigstens eine Rippe auf, welche in eine entsprechend geformte Ausnehmung in dem Stabaufnahmeelement eingreift, um auf diese Weise eine Verdrehsicherung des Halteelements gegenüber dem Stabaufnahmeelement bereitzustellen.

Vorteilhafterweise weist das Halteelement eine halbschalenförmige Ausnehmung zur Lagerung des stabförmigen Elements auf, durch welche eine großflächige Auflagefläche zwischen Halteelement und stabförmigem Element bereitgestellt wird, welche die Kraftübertragung begünstigt.

Vorzugsweise ist das Halteelement in axialer Richtung zwischen einem Anschlag des Stabaufnahmeelements und einem Befestigungsring fixiert. Besonders bevorzugt weist der Befestigungsring auf seiner Innenfläche zumindest abschnittsweise umlaufend wenigstens eine Ausnehmung auf, so dass die Kontaktfläche zwischen dem Halteelement und dem Befestigungsring verringert wird, um dadurch die Reibung zwischen den beiden Elementen zu verringern, die Haltekraft jedoch zu erhöhen.

Vorzugsweise ist der Befestigungsring über ein Gewinde in dem Stabaufnahmeelement fixiert und vorteilhafterweise dort verschweißt, um ein Lösen des Befestigungsrings aus dem Stabaufnahmeelement zu vermeiden.

Die Erfindung wird anhand der folgenden Figuren ausführlich erläutert.

Es zeigt:

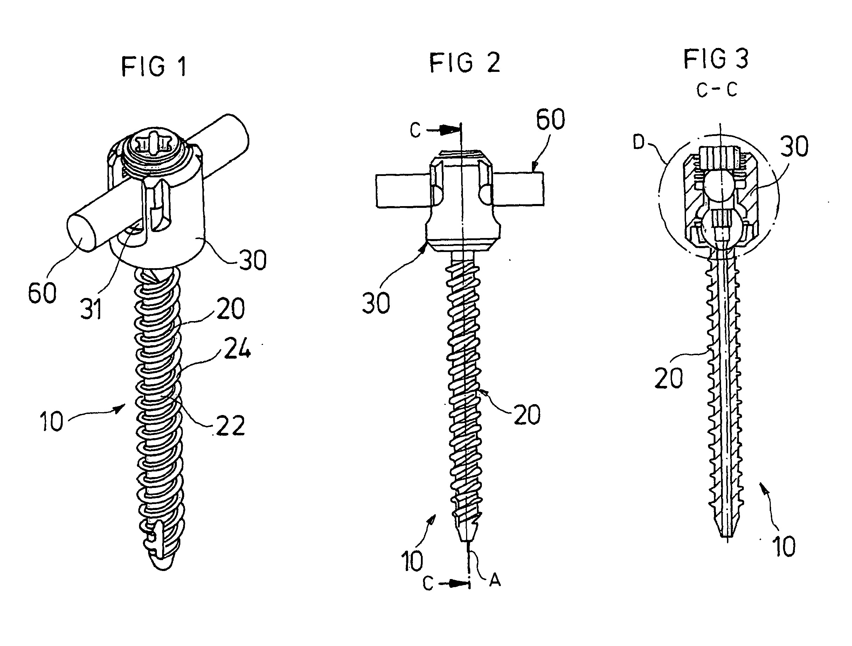

- Fig. 1: eine perspektivische Ansicht eines Knochenveranke- rungselements mit eingesetztem stabförmigen Element,
- Fig. 2: eine Seitenansicht des Knochenverankerungselements gemäß Fig. 1,
- Fig. 3: einen Längsschnitt durch das Knochenverankerungsele- ments gemäß Fig. 1,

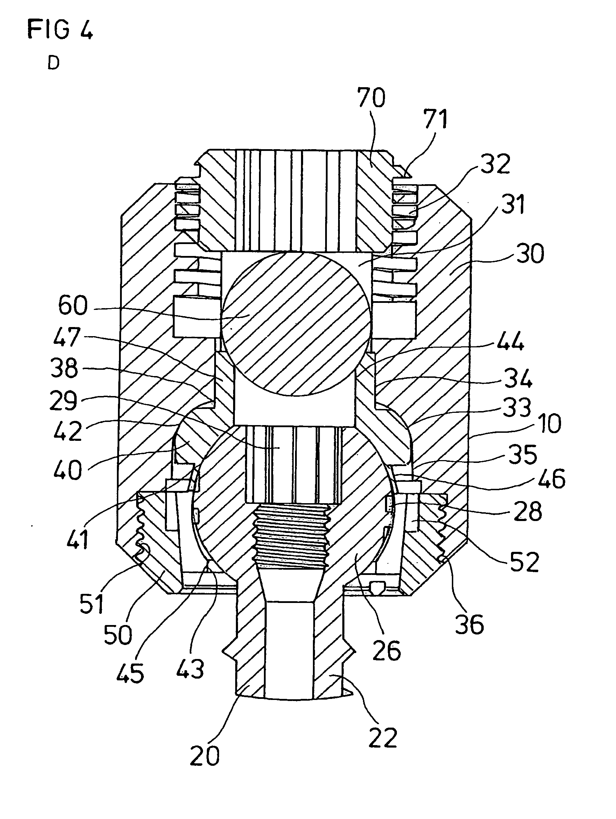

- Fig. 4: eine Ausschnittsvergrößerung aus Fig. 3,

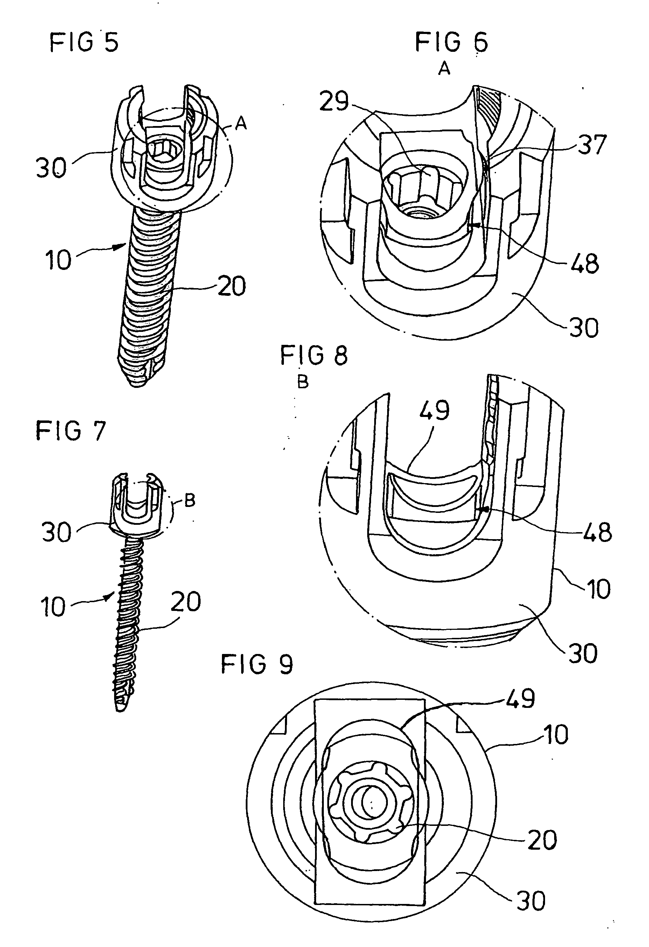

- Fig. 5: eine perspektivische Ansicht des Knochenveranke- rungselements gemäß Fig. 1,
- Fig. 6: eine Ausschnittsvergrößerung aus Fig. 5,
- Fig. 7: eine weitere perspektivische Ansicht des Knochenver- ankerungselements gemäß Fig. 1,
- Fig. 8: eine Ausschnittsvergrößerung aus Fig. 7, und
- Fig. 9: eine Draufsicht auf das Knochenverankerungselements gemäß Fig. 7.

Die Figuren zeigen verschiedene Ansichten eines Ausführungsbeispiels eines Knochenverankerungselements 10, wobei gleiche Bezugszeichen stets gleiche Teile bezeichnen und zur besseren Übersicht nicht sämtliche Bezugsziffern in sämtlichen Figuren angegeben sind.

Das Knochenverankerungselement 10 weist eine Schraube 20 und ein Stabaufnahmeelement 30 zum Verbinden der Schraube 20 mit einem stabförmigen Element 60 auf.

Die Schraube 20 weist einen Schaft 22 und einen daran angeordneten Kopf 26 auf, wobei der Schaft einen Knochgewindeabschnitt 24 und der Kopf zumindest abschnittsweise auf seiner Außenseite einen Gewindeabschnitt 28 aufweist. In den Kopf 26 ist in Längsrichtung eine Ausnehmung 29 eingelassen, in welche zum Eindrehen der Schraube 20 in einem Knochen, beispielsweise in einen Wirbelkörper, ein entsprechendes Werkzeug eingesetzt werden kann.

Das Stabaufnahmeelement 30 weist eine Längsachse A und eine Durchgangsöffnung in Richtung der Längsachse A auf, die auf einer Seite des Stabaufnahmeelements 30, insbesondere der Oberseite des Stabaufnahmeelements 30, in einem quer zur Längsachse A des Stabaufnahmeelements 30 verlaufenden Kanal 31 und auf der gegenüberliegenden Seite des Stabaufnahmeelements 30, insbesondere der Unterseite des Stabaufnahmeelements 30, in einer Halteelementaufnahme 33 zur Aufnahme eines Halteelements 40 mündet. Ausgehend von der Oberseite des Stabaufnahmeelements 30 ist in dem Kanal 31 ein Gewinde 32 angeordnet. Ausgehend von der Unterseite des Stabaufnahmeelements 30 ist ein weiteres Gewinde 36 angeordnet, in welchem ein Befestigungsring 50 mit einem auf dessen Außenseite angeordneten Gewinde 51 zur Befestigung des Halteelements 40 in der Halteelementaufnahme 33 eingesetzt werden kann.

Das Halteelement 40 weist eine Innenfläche 41 und eine Außenfläche 42 auf, wobei die Innenfläche 41 zumindest abschnittsweise hohlkugelig ausgebildet ist, während die Außenfläche 42 im wesentlichen konisch ausgebildet ist. Die Außenfläche 42 des Halteelements 40 weist eine Neigung gegenüber der Längsachse A von etwa 4° auf. In der hohlkugeligen Innenfläche 41 kommt im montierten Zustand der Kopf 26 der Schraube 20 zum Liegen.

Dabei entsprechen sich im wesentlichen die Krümmungen der hohlkugeligen Innenfläche 41 des Halteelements 40 und des Kopfes 26 der Schraube 20, um eine möglichst große Anlagefläche des Kopfes 26 in dem Halteelement 40 bereitzustellen. Die Innenfläche 41 des Halteelements 40 umgreift in montiertem Zustand den Kopf 26 der Schraube 20, wodurch der Kopf 26 der Schraube 20 in axialer Richtung fixiert wird.

Das Halteelement 40 weist insbesondere eine Durchgangsöffnung mit einer ersten Öffnung 43 und einer zweiten Öffnung 44 auf, wobei in montiertem Zustand die erste Öffnung 43 unterhalb, die zweite Öffnung 44 oberhalb des größten Durchmessers der Schraube 20 quer zur Längsrichtung der Schraube 20 verläuft. Bei montiertem Zustand ragt der Schaft 22 der Schraube 20 durch die erste Öffnung 43 des Halteelements 40.

Um den Kopf 26 der Schraube 20 in das Halteelement 40 einbringen zu können, schließt sich an die erste Öffnung 43 auf der Innenfläche 41 des Halteelements 40 ein Gewindeabschnitt 45 an, in welchen der Kopf 26 über seinen Gewindeabschnitt 28 eingedreht werden kann, soweit bis der Kopf 26 innerhalb des Halteelements 40 zu Liegen kommt. Um das Haltelement 40 mit einer Federwirkung auszustatten, ist etwa auf halber Höhe des Halteelements 40 eine umlaufende Ausnehmung 46, insbesondere ein Freistich angeordnet. Dieser begünstigt das Eindrehen des Kopfes 26 der Schraube 20 in das Halteelement 40.

Das Halteelement 40 wird von der Unterseite des Stabaufnahmeelements 30 in die Halteelementaufnahme 33 eingesetzt und nach Einsetzen mittels des Befestigungsrings 50 in dem Stabaufnahmeelement 30 fixiert. Insbesondere wird der Befestigungsring 50 anschließend mit dem Stabaufnahmeelement 30 verschweißt. Der Befestigungsring 50 und das Stabaufnahmeelement 30 sind derart aufeinander abgestimmt, dass die Innenfläche des Befestigungsrings 50 einen konischen Abschnitt 35 der Halteelementaufnahme 33 fortsetzt derart, um eine im wesentlich ebene Anlagefläche für die konische Außenfläche 42 des Halteelements 40 zu bieten. Um die Reibung zwischen dem Befestigungsring 50 und dem Halteelement 40 zu verringern, weist der Befestigungsring 50 vorzugsweise auf seiner Innenfläche eine umlaufende Ausnehmung 52, insbesondere nach Art eines Freistichs, auf, so dass in diesem Bereich ein Leerraum gebildet wird.

Zur zuverlässigen axialen Ausrichtung des Halteelements 40 in dem Stabaufnahmeelement 30 ist zwischen der Halteelementaufnahme 33 und dem Kanal 31 des Stabaufnahmeelements 30 ein zylindrischer Abschnitt 34 kleineren Durchmessers als der konische Abschnitt 35 des Stabaufnahmeelements 30 angeordnet, in welchen ein an dem Halteelement 40 angeordneter zylindrischer Abschnitt 47 im wesentlichen formflüssig eingreift. Um ein Verdrehen um die Längsachse A des Halteelements 40 in dem Stabaufnahmeelement 30 zu verhindern, ist an dem zylindrischen Abschnitt 47 eine längsverlaufenden Rippe 48 angeordnet, die in eine entsprechende Ausnehmung 37 des Stabaufnahmeelements 30 eingreift.

Das Halteelement 40 wird bis zu einem Anschlag 38 in die Halteelement 33 des Stabaufnahmeelements 30 eingeschoben und ist so zwischen dem Befestigungsring 50 und dem Anschlag 38 des Stabaufnahmeelements 30 in axialer Richtung fixiert. Der Anschlag 38 wird dabei insbesondere durch den Übergang zwischen dem konischen Abschnitt 35 und dem zylindrischen Abschnitt 34 der Halteelementaufnahme 33 gebildet, an welchem sich der Durchmesser der Durchgangsöffnung durch das Stabaufnahmeelement 30 von dem zylindrischen Abschnitt 34 ausgehend erweitert.

Das Stabaufnahmeelement 30 einschließlich des über den verschweißten Befestigungsring 50 darin fixierten Halteelements 40 kann auch nach Platzieren der Schraube 20 in dem Knochen auf den Kopf 26 der Schraube 20 aufgedreht werden, wobei die kugelige Innenfläche 41 des Halteelements 40 ein Verkippen der Längsachse des Stabaufnahmeelements 30 gegenüber der Längsachse der Schraube 20 zulässt.

Nach Aufbringen des Stabaufnahmeelements 30 auf die Schraube 20 wird das stabförmige Element 60 in den Kanal 31 eingelegt. Das Halteelement 40 weist auf seiner dem Kanal 31 zugewanden Seite eine halbschalenförmige Ausnehmung 47 auf, in welche das stabförmigen Element 60 im wesentlichen formschlüssig eingelegt werden kann, um die Auflagefläche des stabförmigen Elements 60 in dem Halteelement 40 zu vergrößern. Das stabförmige Element 60 wird anschließend in dem Kanal 31 über ein Sicherungselement 70 fixiert, welches mit einem Außengewinde 71 in das Gewinde 32 des Stabaufnahmeelements 30 eingeschraubt wird soweit, bis das stabförmige Element 60 in axialer Richtung zwischen dem Sicherungselement 70 und dem Halteelement 40 fixiert ist.

### Bezugszeichenliste

- 10: Knochenverankerungselement

- 20: Schraube
- 22: Schaft
- 24: Knochengewindeabschnitt
- 26: Kopf
- 28: Gewindeabschnitt
- 29: Ausnehmung

- 30: Stabaufnahmeelement
- 31: Kanal
- 32: Gewinde
- 33: Halteelementaufnahme
- 34: zylindrischer Abschnitt
- 35: konischer Abschnitt
- 36: Gewinde
- 37: Ausnehmung
- 38: Anschlag

- 40: Halteelement
- 41: Innenfläche
- 42: Außenfläche
- 43: erste Öffnung
- 44: zweite Öffnung
- 45: Gewindeabschnitt
- 46: Ausnehmung
- 47: zylindrischer Abschnitt
- 48: Rippe
- 49: Ausnehmung

- 50: Befestigungsring
- 51: Gewinde
- 52: Ausnehmung

- 60: stabförmiges Element

- 70: Sicherungselement
- 71: Außengewinde

- A: Längsachse

## Patentansprüche

1. Knochenverankerungselement (10) mit einer einen Schaft (22) und einen abschnittsweise kugeligen Kopf (26) aufweisenden Schraube (20) und einem eine Längsachse (A) aufweisenden Stabaufnahmeelement (30) zum Verbinden der Schraube (20) mit einem stabförmigen Element (60), wobei der Schaft (22) einen Knochengewindeabschnitt (24) und der Kopf 26) einen Gewindeabschnitt (28) aufweist, wobei das Stabaufnahmeelement (30) quer zur Längsachse (A) einen Kanal (31) zur Aufnahme des stabförmigen Elements (60) und ein Halteelement (40) mit einer abschnittsweise hohlkugelig ausgebildeten Innenfläche (41) zur Aufnahme des Kopfs (26) der Schraube (20) aufweist, wobei
das Halteelement (40) mit seiner hohlkugeligen Innenfläche (41) den Kopf (26) der Schraube (20) zumindest teilweise, d. h. sowohl oberhalb als auch unterhalb eines größten Durchmessers des Kopfes (26) der Schraube (20) quer zur Längsachse der Schraube (20) umgreift, und wobei der Kopf (26) der Schraube (20) mittels seines Gewindeabschnittes (28) in eine erste Öffnung (43) des Halteelements (40) eindrehbar ist, bis der Kopf (26) in der hohlkugeligen Innenfläche (41) des Halteelements (40) zu liegen kommt,**dadurch gekennzeichnet, dass** das Halteelement (40) mit einer Federwirkung ausgestattet ist, dass das Halteelement (40) eine abschnittsweise konisch ausgebildete Außenfläche (42) aufweist, und dass das Stabaufnahmeelement (30) einen konischen Abschnitt (35) als Anlagefläche für die konische Außenfläche (42) des Halteelements (40) aufweist.

2. Knochenverankerungselement nach Anspruch 1,
**dadurch gekennzeichnet, dass** sich an die erste Öffnung (43) auf der Innenfläche (41) des Halteelements (40) ein Gewindeabschnitt (45) anschließt.

3. Knochenverankerungselement nach einem der vorhergehenden Ansprüche,
**dadurch gekennzeichnet, dass** auf der Außenfläche (42) des Halteelements (40) zumindest abschnittsweise umlaufend wenigstens eine Ausnehmung (46) angeordnet ist.

4. Knochenverankerungselement nach einem der vorhergehenden Ansprüche,
**dadurch gekennzeichnet, dass** auf der Außenfläche (42) des Halteelements (40) umlaufend ein Freistich angeordnet ist.

5. Knochenverankerungselement nach einem der vorhergehenden Ansprüche,
**dadurch gekennzeichnet, dass** das Halteelement (40) einen zylindrischen Abschnitt (47) aufweist.

6. Knochenverankerungselement nach Anspruch 5,
**dadurch gekennzeichnet, dass** das Halteelement (40) in Längsrichtung wenigstens eine Rippe (48) aufweist, welche in eine entsprechend geformte Ausnehmung (37) in dem Stabaufnahmeelement (30) eingreift.

7. Knochenverankerungselement nach einem der vorhergehenden Ansprüche,
**dadurch gekennzeichnet, dass** das Halteelement (40) eine halbschalenförmige Ausnehmung (49) zur Lagerung des stabförmigen Elements (60) aufweist.

8. Knochenverankerungselement nach einem der vorhergehenden Ansprüche,
**dadurch gekennzeichnet, dass** das Halteelement (40) in axialer Richtung zwischen einem Anschlag (38) des Stabaufnahmeelements (30) und einem Befestigungsring (50) fixiert ist.

9. Knochenverankerungselement nach Anspruch 8,
**dadurch gekennzeichnet, dass** der Befestigungsring (50) auf seiner Innenfläche zumindest abschnittsweise umlaufend wenigstens eine Ausnehmung (52) aufweist.

10. Knochenverankerungselement nach Anspruch 8,
**dadurch gekennzeichnet, dass** der Befestigungsring (50) über ein Gewinde (51) in dem Stabaufnahmeelement (30) fixiert und vorzugsweise verschweißt ist.

## Claims

1. A bone-anchoring element (10) with a screw (20) having a shank (22) and a locally spherical head (26) and with a rod-receiving element (30) having a longitudinal axis (A) for connecting the screw (20) to a rod-shaped element (60), wherein the shank (22) has a bone thread portion (24) and the head (26) has a thread portion (28), wherein the rod-receiving element (30) has transversely to the longitudinal axis (A) a channel (31) for receiving the rod-shaped element (60) and a holding element (40) with an inner face (41) designed locally in the shape of a hollow sphere for receiving the head (26) of the screw (20), wherein the holding element (40) engages with its inner face (41) in the shape of a hollow sphere around the head (26) of the screw (20) at least in part, i.e. both above and below a maximum diameter of the head (26) of the screw (20) transversely to the longitudinal axis of the screw (20), and wherein the head (26) of the screw (20) is capable of being screwed by means of its thread portion (28) into a first opening (43) in the holding element (40) until the head (26) comes to rest in the inner face (41) of the holding element (40) in the shape of a hollow sphere, **characterized in that** the holding element (40) is provided with a spring action, the holding element (40) has an outer face (42) designed conically locally, and the rod-receiving element (30) has a conical portion (35) as an abutment face for the conical outer face (42) of the holding element (40).

2. A bone-anchoring element according to claim 1, **characterized in that** the first opening (43) on the inner face (41) of the holding element (40) is adjoined by a thread portion (45).

3. A bone-anchoring element according to one of the preceding claims, **characterized in that** at least one recess (46) is provided in a peripheral manner at least locally on the outer face (42) of the holding element (40).

4. A bone-anchoring element according to any one of the preceding claims, **characterized in that** an undercut is provided on the outer face (42) of the holding element (40).

5. A bone-anchoring element according to any one of the preceding claims, **characterized in that** the holding element (40) has a cylindrical portion (47).

6. A bone-anchoring element according to claim 5, **characterized in that** in the longitudinal direction the holding element (40) has at least one rib (48) which engages in a correspondingly shaped recess (37) in the rod-receiving element (30).

7. A bone-anchoring element according to any one of the preceding claims, **characterized in that** the holding element (40) has a recess (49) in the shape of a half shell for mounting the rod-shaped element (60).

8. A bone-anchoring element according to any one of the preceding claims, **characterized in that** the holding element (40) is fixed in the axial direction between a stop (38) of the rod-receiving element (30) and a fastening ring (50).

9. A bone-anchoring element according to claim 8, **characterized in that** the fastening ring (50) has at least one recess (52) in a peripheral manner at least locally on its inner face.

10. A bone-anchoring element according to claim 8, **characterized in that** the fastening ring (50) is fixed in the rod-receiving element (30) by way of a thread (51) and is preferably welded.

## Revendications

1. Elément d'ancrage dans un os (10) comportant une vis (20) comprenant une tige (22) et une tête (26) à segments sphériques ainsi qu'un élément de réception d'une barre (30) d'axe longitudinal (A) pour relier la vis (20) avec un élément en forme de barre (60), la tige (22) comportant une partie filetée à visser dans un os (24) et la tête (26) comportant une partie filetée (28),
l'élément de réception d'une barre (30) comportant, transversalement à l'axe longitudinal (A) un canal (31) de réception de l'élément en forme de barre (60) et un élément de retenue (40) avec une surface interne (41) réalisée par segments en forme de sphère creuse pour la réception de la tête (26) de la vis (20),
l'élément de retenue (40) venant en prise, transversalement à l'axe longitudinal de la vis (20), et par sa surface interne (41) en forme de sphère creuse avec la tête (26) de la vis (20), ce au moins partiellement, c'est-à-dire aussi bien au-dessus qu'en dessous du plus grand diamètre de cette tête (26) de la vis (20), et
la tête (26) de la vis (20) pouvant être vissée par sa partie filetée (28) dans une première ouverture (43) de l'élément de retenue (40) jusqu'à ce qu'elle soit située dans la surface interne (41) en forme de sphère creuse de l'élément de retenue (40),
**caractérisé en ce que**
- l'élément de retenue (40) présente une élasticité,
- l'élément de retenue (40) comporte une surface externe (42) réalisée par segments en forme de cône, et
- l'élément de réception d'une barre ou de barreau (30) comporte une partie conique (35) constituant une surface d'appui pour la surface externe conique (42) de l'élément de retenue (40).

2. Elément d'ancrage dans un os conforme à la revendication 1,
**caractérisé en ce que**
une partie filetée (45) se raccorde à la première ouverture (43) sur la surface interne (41) de l'élément de retenue (40).

3. Elément d'ancrage dans un os conforme à l'une des revendications précédentes,
**caractérisé en ce que**
la surface extérieure (42) de l'élément de retenue (40) comporte au moins un évidement (46) au moins par segments périphériques.

4. Elément d'ancrage dans un os conforme à l'une des revendications précédentes,
**caractérisé en ce que**
la surface extérieure (42) de l'élément de maintien (40) comporte une rainure de dégagement périphérique.

5. Elément d'ancrage dans un os conforme à l'une des revendications précédentes,
**caractérisé en ce que**
l'élément de retenue (40) comporte une partie cylindrique (47).

6. Elément d'ancrage dans un os conforme à la revendication 5,
**caractérisé en ce que**
l'élément de retenue (40) comporte en direction longitudinale au moins une nervure (48) qui vient en prise dans un évidement (37) de forme correspondante réalisé dans l'élément de réception d'une barre (30).

7. Elément d'ancrage dans un os conforme à l'une des revendications précédentes,
**caractérisé en ce que**
l'élément de retenue (40) comporte un évidement (49) en forme de demi-coquille pour le positionnement de l'élément de forme de barre (60).

8. Elément d'ancrage dans un os conforme à l'une des revendications précédentes,
**caractérisé en ce que**
l'élément de retenue (40) est fixé en direction axiale entre une butée (38) de l'élément de réception d'une barre (30) et une bague de fixation (50).

9. Elément d'ancrage dans un os conforme à la revendication 8,
**caractérisé en ce que**
la bague de fixation (50) comporte, sur sa surface interne au moins un évidement (52) au moins par segments périphériques.

10. Elément d'ancrage dans un os conforme à la revendication 8,
**caractérisé en ce que**
la bague de fixation (50) est fixée dans l'élément de réception d'une barre (30) par un filetage (51) et est de préférence soudée.
